# EUROPÄISCHE PATENTSCHRIFT

(11) **EP 1 658 014 B1**
(45) Veröffentlichungstag und Bekanntmachung des Hinweises auf die Patenterteilung: **01.05.2013**
(21) Anmeldenummer: 04763398.7
(22) Anmeldetag: 22.07.2004
(51) Int. Cl.: A61B 17/72

(54) **VORRICHTUNG ZUM VERLÄNGERN VON KNOCHEN**
DEVICE FOR EXTENDING BONES
DISPOSITIF D'ALLONGEMENT DES OS

(30) Priorität: 28.08.2003 DE 10340025
(43) Veröffentlichungstag der Anmeldung: 24.05.2006
(73) Patentinhaber: Wittenstein AG, 97999 Igersheim (DE)
(72) Erfinder: STAUCH, Roman, 97959 Assamstadt (DE)
(74) Vertreter: Weiss, Peter
(86) Internationale Anmeldenummer: PCT/EP2004/008194
(87) Internationale Veröffentlichungsnummer: WO 2005/027762

(56) Entgegenhaltungen:
- WO-A-01/78614
- WO-A-98/30163
- WO-A1-02/094113
- FR-A1- 2 726 757
- US-A- 5 601 551
- US-A- 5 704 938

## Beschreibung

Die vorliegende Erfindung betrifft eine Vorrichtung zum Verlängern von Knochen nach dem Oberbegriff des Anspruchs 1.

Derartige Vorrichtungen sind in vielfältiger Form und Ausführungen auf dem Markt bekannt und gebräuchlich. Sie dienen insbesondere zur Distraktion von beliebigen Knochen. Diese können in eine Knochenhöhle bzw. in einen Markraum eines Knochens eingesetzt werden, wobei nach einem Durchtrennen eines Knochens eine Distraktion erfolgen kann.

Nachteilig ist bei herkömmlichen Vorrichtungen, dass diese keine hohen Distraktionskräfte aufweisen, apparativ sehr aufwendig und kostenintensiv herzustellen sind und zudem in einer begrenzten Einbaugrösse herstellbar sind. Daher sind die Einsatzmöglichkeiten, insbesondere bei klein ausgebildeten Knochen hinsichtlich Länge und Durchmesser sehr beschränkt.

Zudem ist nachteilig, dass herkömmliche Distraktionseinrichtungen einen geringen Hub aufweisen und nach Ausschöpfung des vollständigen Hubes, andere Distraktionsvorrichtungen oder -einrichtungen eingesetzt werden müssen, was ebenfalls unerwünscht ist.

Der vorliegenden Erfindung liegt die Aufgabe zugrunde, eine Vorrichtung der eingangs genannten Art zu schaffen, welche die genannten Nachteile beseitigt, und mit welcher eine Vorrichtung geschaffen wird, die hohe Distraktionskräfte zulässt und einen sehr hohen Hub aufweist.

Zudem soll eine derartige Vorrichtung in allen möglichen Grössen für beliebige Einbauräume herzustellen und einzusetzen sein. Zudem soll eine problemlose, berührungslose Einspeisung von Energie- und Datenübertragung sowie Ansteuerung möglich sein.

Die WO 98/30163 A offenbart eine Distraktionsvorrichtung nach dem Oberbegriff des Anspruchs 1 zum Auseinanderbewegen zweier Knochenteile, wobei koaxial ineinander zwei schiebbar gelagerte zylindrische Hülsen mittels eines Planetenrollsystems auseinanderbewegbar sind.

Die US 5,704,938 offenbart eine Distraktionsvorrichtung für Konchen zum Implantieren in einen Knochen.

Die US 5,601,551 offenbart eine Distraktionsvorrichtung, bei welcher auf einem starren Balken mehrere Bauteile einzeln hin- und herbewegbar angeordnet sind, wobei die Distraktionsvorrichtung ausserhalb eines Knochens angreift. Die DE 198 29 523 A1 offenbart eine Distraktionsvorrichtung mit zwei axial gegeneinander bewegbaren Elementen.

Weiter wird auf die FR 2 726 757 und die WO02/094113 A1 hingewiesen, welche ebenfalls Knochenverlängerungsvorrichtungen beschreiben.

Zur Lösung dieser Aufgabe führt der Gegenstand des Anspruchs 1.

Bei der vorliegenden Erfindung hat sich als besonders vorteilhaft erwiesen, dass das zweite Element gegenüber dem ersten Element radial verdrehsicher in dieses eingesetzt ist. Die Verdrehsicherung kann auf unterschiedliche Weise erfolgen. Ein Aussenquerschnitt des zweiten Elementes ist polygonartig ausgebildet, und ein entsprechend ausgebildetes Führungselement mit entsprechend ausgebildetem Innenquerschnitt des ersten Elementes ggf. im endseitigen Bereich gewährleistet eine entsprechend radiale Verdrehsicherung. Hierdurch wird ermöglicht, dass innerhalb des ersten Elementes mittels eines Planetenrollensystems oder Gewinde- oder Spindelsystem oder ähnlichen mittels des Antriebselementes eine Distraktion, insbesondere eine axiale Bewegung des zweiten Elementes gegenüber dem ersten Element gewährleitstet wird. Diese Distraktion in Abhängigkeit der Übersetzungsverhältnisse des Planetenrollensystems gewährleistet sehr hohe Distraktionskräfte, wobei auch gleichzeitig eine exakte Wegstrecke bzw. Distraktion je Zeiteinheit verfahrbar ist. Zudem ist exakt die Distraktion bzw. jeder Hub ansteuerbar und verfahrbar.

Zusätzlich können entsprechende im Element und/oder zwischen einer Welle und dem Antriebselement, einem Elektromotor eingesetzte Kraftsensoren das Distraktionsverhalten aufgrund der anliegenden Kräfte als Druckkräfte oder Drehmomente bestimmen.

Auf diese Weise lässt sich auch, insbesondere bei der Distraktion von Knochen der Distraktionsprozess exakt steuern, regeln und überwachen. Zudem ist auch eine kraftgesteuerte Distraktion bzw. axiale Bewegung der beiden Elemente auseinander möglich. Dies soll ebenfalls im Rahmen der vorliegenden Erfindung liegen.

Ferner ist dem als Aufnahmehülse ausgebildeten ersten Element ein Elektromotor, und ggf. eine Elektronikeinheit und daran anschliessend ein Energie- und/oder Datenübertragungselement zugeordnet. Vorzugsweise im endseitigen Bereich der beiden Elemente sind radiale Verriegelungsbohrungen vorgesehen, um die Vorrichtung, insbesondere den als Vorrichtung ausgebildeten Marknagel im Knochen bspw. in einen Röhrenknochen, über hier nicht dargestellte Befestigungselemente, Schrauben, Nägel od. dgl. festzulegen.

Bevorzugt erfolgt die Ansteuerung und Versorgung berührungslos und induktiv über die Energie- und/oder Datenübertragungselemente.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 einen schematisch dargestellten Teillängsschnitt durch das erste Element im endseitigen Bereich;
Figur 2a einen schematisch dargestellten Teillängsschnitt durch das erste Element gemäss Figur 1;
Figur 2b einen schematisch dargestellten Querschnitt durch das zweite Element gemäss Figur 1;
Figur 3 einen schematisch dargestellten Querschnitt durch die Elemente 1 und 2 als weiteres Ausführungsbeispiel.

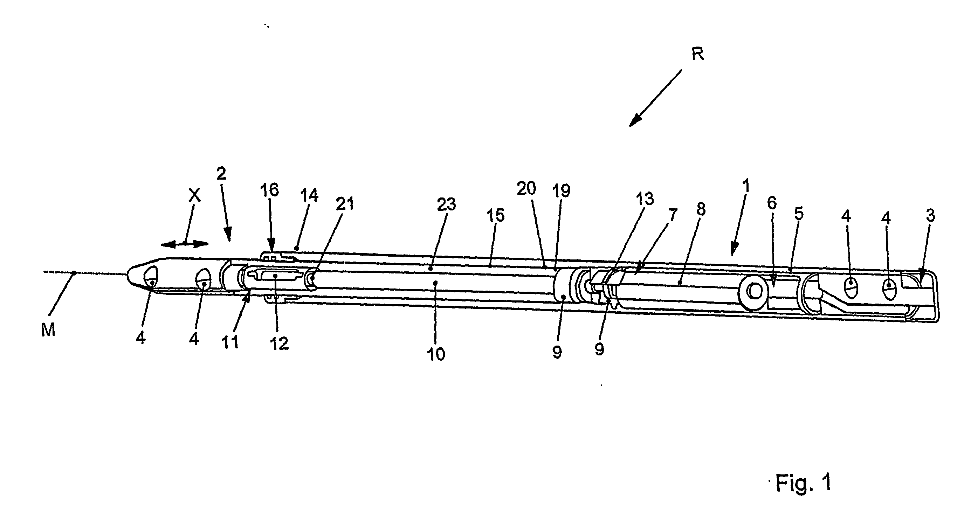

Gemäss Figur 1 weist eine erfindungsgemässe Vorrichtung R ein erstes Element 1 und ein darin linear und axial geführtes zweites Element 2 auf.

Im ersten Element 1, insbesondere im endseitigen Bereich ist integriert ein Energie- und/oder Datenübertragungselement 3 eingesetzt, welches die entsprechende Energie liefert und einen Datenaustausch bidirektional berührungslos gewährleistet. Im endseitigen Bereich des Elements 1 sind ferner zumindest zwei radiale Verriegelungsbohrungen 4 vorgesehen, die einem Festlegen der Vorrichtung R bspw. in einem zu verlängernden Knochen dienen. Gleichzeitig wird eine radiale Verdrehsicherung des Elementes 1 gegenüber dem Knochen beim Festlegen gewährleistet.

Das Element 1 ist als Aufnahmehülse 5 ausgebildet, die eine hier nur angedeutete Elektronikeinheit 6 aufweist, die mit dem Energie- und/oder Datenübertragungselement 3 sowie auch einem Antriebselement 7 in Verbindung steht.
Das Antriebselement 7 umfasst einen Elektromotor 8, der über hier nur angedeutete Lagerungen 9 eine Antriebswelle 10 rotativ in Bewegung versetzt. An die Antriebswelle 10 schliesst endseits ein Planetenrollensystem 11 an, in welchem eine Mehrzahl von hier nicht näher dargestellten Planeten vorgesehen sind, die über die Antriebswelle 10 und den hier nur angedeuteten Plantenradträger 12 angetrieben werden.

Vorzugsweise schliesst an den Elektromotor 8 ein Kraftsensor 13 zur Bestimmung der axialen Kräfte der Welle sowie auch der Drehmomente an, der wiederum mit der Elektronikeinheit 6 in Verbindung steht.

Zwischen dem Elektromotor 8 und einem endseitigen Bereich 14 des ersten Elementes 1 weist dieses im Inneren einen Führungsbereich 15 auf, welcher in einer nicht erfindungsgemäßen Ausführung zylindrisch ausgebildet ist.

Im endseitigen Bereich 14 ist dem Element 1 ein Führungselement 16 aufgesetzt, welches einen Innenquerschnitt 17 aufweist, das einem Aussenquerschnitt 18 des zweiten Elementes 2 in etwa entspricht.

Erfindungsgemäß sind Innenquerschnitt 17 und Aussenquerschnitt 18 querschnittlich polygonartig ausgebildet. Auf diese Weise wird verhindert, dass sich das Element 2, welches im Führungsbereich 15 des Elementes 1 geführt ist, radial verdrehen kann. Es kann sich zwar axial entlang einer Mittelachse M hin und her bewegen, jedoch nicht radial verdrehen.

Vorzugsweise ist das Element 2 nahezu vollständig, als Polygonprofil betreffend seines Aussenquerschnittes ausgebildet.

Jedoch in seinem endseitigen Bereich 19 kann dieses eine querschnittlich andere Aussenkontur 20 aufweisen, die dem Führungsbereich 15 des ersten Elementes 1 in etwa entspricht.

Das Innere des Elementes 2 ist vorzugsweise als Gewinde ausgebildet, welches mit dem Planetenrollensystem 11, Spindelsystem oder ähnlichen bzw. dessen hier nicht näher dargestellten Planeten zusammenwirkt.

Durch entsprechendes Antreiben der Antriebswelle 10 bzw. des Planetenrollensystems 11, lässt sich entlang einer in Figur 1 angedeuteten Mittelachse M in dargestellter Doppelpfeilrichtung X das Element 2 aus dem Element 1 herausbewegen bzw. herausfahren.

Das Element 2 kann aus dem Element 1 axial herausbewegt werden, bis der endseitige Bereich 19 des Elementes 2 innen am Führungselement 16 anschlägt.

Auf diese Weise lässt sich ein sehr grosser Hub des Elementes 2 gegenüber dem Element 1 gewährleisten.

Wichtig ist bei der vorliegenden Ausführung der Erfindung, dass der grosse Hub auch dadurch erreicht werden kann, dass über das Planetenrollensystem 11 das Element 2 gegenüber dem Element 1 axial unter sehr hohen Kräften absolut präzise herausbewegt werden kann, wobei das Element 2 radial verdrehfest gegenüber dem Element 1 über das Führungselement 16 geführt ist.

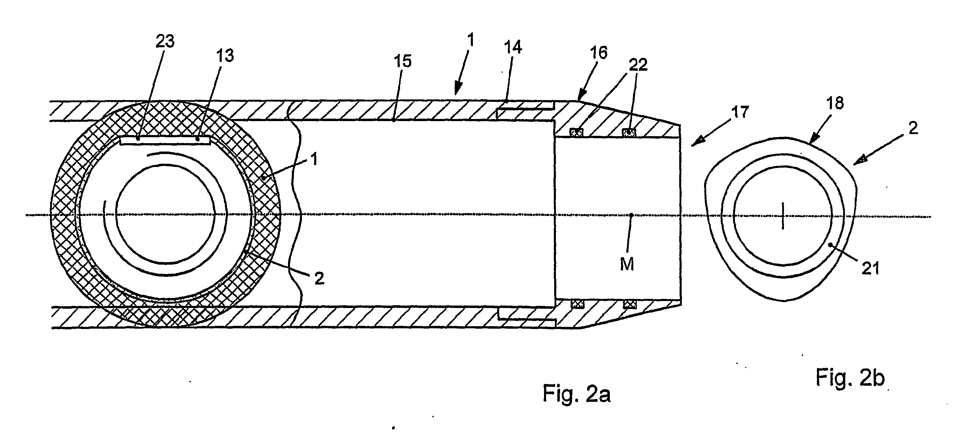

Das Führungselement 16 kann ein oder mehrere Dichtelemente 22, wie es in den Figuren 1 und 2a angedeutet ist, aufweisen. Diese dienen der Abdichtung der Elemente 1 und 2 gegeneinander im endseitigen Bereich 14.

Nach einer nicht erfindungsgemäßen Ausführung wird das Element 2 nicht innerhalb des Elementes 1 geführt, sondern dieses bspw. als aussenliegende Hülse übergreift bzw. das Element 1 darin aufnimmt und verdrehsicher führt. In diesem Fall kann bspw. der Planetenradträger 12 ausserhalb des endseitigen Bereiches 14 des Elementes 1 liegen und ein entsprechendes inneres Gewinde 21 des Elementes 2 kämmen.

Ferner soll im Rahmen der vorliegenden Erfindung liegen, dass bspw. der endseitige Bereich 19 des Elementes 2 querschnittlich rund, polygonartig, mehreckartig oder anderswie ausgeformt sind, um eine axiale und radiale Führung gegenüber dem Element 1 zu gewährleisten wobei nicht zwangsweise eine Verdrehsicherung hier gegeben sein muss, da die radiale Verdrehsicherung über das Führungselement 16 zwischen Element 1 und 2 auch erfolgen kann.

Zwischen dem endseitigen Bereich 19 und dem Führungselement 16 kann innerhalb des Führungsbereiches 15 noch ein Aufnahmeraum 23 zur Unterbringung von Sensoren, Kraftsensoren, Wegsensoren od. dgl. vorgesehen sein, wie es bspw. auch in Figur 2a angedeutet ist.

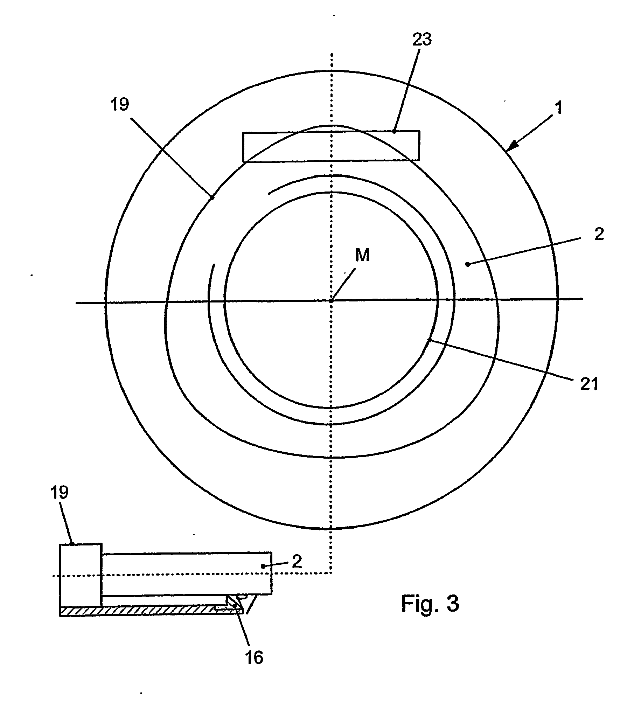

In dem Ausführungsbeispiel der vorliegenden Erfindung gemäss Figur 3 ist ein Aufnahmeraum 23 angedeutet, wobei auch hier daran gedacht sein kann, dass lediglich der endseitige Bereich 19 des Elementes 2 als Polygonprofil ausgebildet sein kann, welches entsprechend die radiale Verdrehsicherung bildet, wobei der innere Führungsbereich 15 des Elementes 1 ebenfalls polygonartig ausgebildet ist. Dabei kann das an den endseitigen Bereich 19 anschliessende Element 2 querschnittlich rund ausgebildet sein. Entsprechend ist dann ein Innenquerschnitt 17 des Führungselementes 16 des ersten Elementes 1 innen rund ausgebildet, und dient lediglich der radialen und axialen Führung. Die Verdrehsicherung erfolgt dann lediglich im endseitigen Bereich 19. Hierauf sei die Erfindung nicht beschränkt.

### Positionszahlenliste

| | | | | | |
|---|---|---|---|---|---|
| 1 | Element | 34 | | 67 | |
| 2 | Element | 35 | | 68 | |
| 3 | Energie- und/oder Datenübertragungselement | 36 | | 69 | |
| 4 | Verriegelungsbohrung | 37 | | 70 | |
| 5 | Aufnahmehülse | 38 | | 71 | |
| 6 | Elektronikeinheit | 39 | | 72 | |
| 7 | Antriebselement | 40 | | 73 | |
| 8 | Elektromotor | 41 | | 74 | |
| 9 | Lagerung | 42 | | 75 | |
| 10 | Antriebswelle | 43 | | 76 | |
| 11 | Planetenrollsystem | 44 | | 77 | |
| 12 | Planetenradträger | 45 | | 78 | |
| 13 | Kraftsensor | 46 | | 79 | |
| 14 | endseitiger Bereich | 47 | | | |
| 15 | Führungsbereich | 48 | | | |
| 16 | Führungselement | 49 | | R | Vorrichtung |
| 17 | Innenquerschnitt | 50 | | | |
| 18 | Aussenqueschnitt | 51 | | X | Doppelpfeilrichtung |
| 19 | endseitiger Bereich | 52 | | | |
| 20 | Aussenkontur | 53 | | | |
| 21 | Gewinde | 54 | | | |
| 22 | Dichtelement | 55 | | | |
| 23 | Aufnahmeraum | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Vorrichtung zum Verlängern von Knochen mit zwei gegeneinander bewegbaren Elementen (1, 2), welche über zumindest ein Antriebselement (7) miteinander in Verbindung stehen, wobei die beiden Elemente (1, 2) beim axialen Gegeneinanderbewegen zueinander verdrehfest geführt sind, und
wobei das Antriebselement dem ersten (1) der beiden Elemente (1, 2) zugeordnet ist, und das erste Element (1) als Aufnahmehülse (5) ausgebildet ist, in welchem einends zumindest eine radiale Verriegelungsbohrung (4) vorgesehen ist, wobei in der Aufnahmehülse (5) das Antriebselement (7) als Elektromotor (8) eingesetzt ist, welches ggf. über eine Antriebswelle (10) oder direkt ein Planetenrollensystem (11) oder ein Gewinde- oder Spindelsystem antreibt,
**dadurch gekennzeichnet, dass**
- ein Innenquerschnitt (17) des ersten Elementes (1) polygonartig oder rechteckartig als Führungsbereich (15) für das zweite Element (2) der beiden Elemente (1, 2) ausgebildet ist; und
- ein Aussenquerschnitt (18) des zweiten Elementes (2) zumindest teilweise dem Innenquerschnitt (17) des ersten Elementes (1) im Führungsbereich (15) entspricht und polygonartig oder rechteckartig ausgebildet ist.

2. Vorrichtung nach Anspruch 1, **dadurch gekennzeichnet, dass** dem Antriebselement (7), dem Elektromotor (8), der Antriebswelle (10) und/oder dem Planetenrollensystem (11) bzw. Spindelsystem zumindest ein Kraftsensor (13) und/oder Wegsensor (13) zugeordnet ist, welcher mit beispielswiese Spindelsystemen dem Antriebselement (7), insbesondere dem Elektromotor (8) mit einer Elektronikeinheit (6) in Verbindung steht.

3. Vorrichtung nach einem der Ansprüche 1 oder 2, **dadurch gekennzeichnet, dass** ein Innenquerschnitt des zweiten Elementes (2) als zylindrische Bohrung ausgebildet ist, die in ihrer inneren Mantelfläche mit einem Gewinde (21) versehen ist, welches mit dem an die Antriebswelle (10) anschliessenden Plantenrollensystem (11) bzw. Gewinde- oder Spindelsystem in Eingriff steht.

4. Vorrichtung nach einem der vorhergehenden Ansprüche, **dadurch gekennzeichnet, dass** endseits das zweite Element (2) zumindest eine radiale Verriegelungsbohrung (4) aufweist.

5. Vorrichtung nach einem der vorhergehenden Ansprüche, **dadurch gekennzeichnet, dass** wenigstens eine Abdichtung oder zumindest ein Dichtelement (22) zwischen den Elementen (1, 2) eingesetzt ist.

6. Vorrichtung nach einem der vorhergehenden Ansprüche, **dadurch gekennzeichnet, dass** dem endseitigen Bereich (14) des ersten Elementes (1) gegenüberliegend ein Energie- und/oder Datenübertragungselement (3), insbesondere zumindest eine Spule zur induktiven Energie- und/oder Datenübertragung, bidirektional und berührungslos eingesetzt ist, die mit dem Antriebselement (7) und/oder der Elektronikeinheit (6) in Verbindung steht.

## Claims

1. A device for lengthening bones, having two elements (1, 2), movable in relation to one another, which are connected to one another via at least one driving element (7), wherein upon axial movement in relation to one another, the two elements (1, 2) are guided in a twist-proof manner with respect to one another, and
wherein the driving element is associated with the first (1) of the two elements (1, 2), and the first element (1) is in the form of a receiving sleeve (5) in which at least one radial locking bore (4) is provided at one end, wherein the driving element (7) is inserted in the receiving sleeve (5) as an electric motor (8) which via a drive shaft (10) or in a direct manner as the case may be drives a planetary roller system (11) or a thread- or spindle system,
**characterised in that**
- an inner cross-section (17) of the first element (1) is polygon-like or rectangle-like in design as a guide region (15) for the second element (2) of the two elements (1, 2); and
- an outer cross-section (18) of the second element (2) at least partially corresponds to the inner cross-section (17) of the first element (1) in the guide region (15) and is polygon-like or rectangle-like in design.

2. A device according to claim 1, **characterised in that** at least one force sensor (13) and/or displacement sensor (13) is associated with the driving element (7), the electric motor (8), the drive shaft (10) and/or the planetary roller system (11) or spindle system, by which sensor (13) with for example spindle systems, the driving element (7), in particular the electric motor (8), is connected to an electronics unit (6).

3. A device according to any one of claims 1 and 2, **characterised in that** an inner cross-section of the second element (2) is in the form of a cylindrical bore provided with a thread (21) in its inner surface, which thread (21) engages the planetary roller system (11) or thread- or spindle system connected to the drive shaft (10).

4. A device according to any one of the preceding claims, **characterised in that** the second element (2) has at least one radial locking bore (4) at the end.

5. A device according to any one of the preceding claims, **characterised in that** at least one sealing or at least one sealing element (22) is inserted between the elements (1, 2).

6. A device according to any one of the preceding claims, **characterised in that** opposite the end region (14) of the first element (1), an energy- and/or data transmission element (3), in particular at least one coil for inductive energy- and/or data transmission, is inserted in a bi-directional and contactless manner, which coil is connected to the driving element (7) and/or the electronics unit (6).

## Revendications

1. Dispositif d'allongement d'os avec deux éléments (1, 2) déplaçables l'un par rapport à l'autre qui communiquent l'un avec l'autre par l'intermédiaire d'au moins un élément d'entraînement (7), les deux éléments (1, 2) étant, lors du déplacement axial l'un par rapport à l'autre, guidés fixes en rotation l'un par rapport à l'autre, et l'élément d'entraînement étant associé au premier (1) des deux éléments (1, 2), et le premier élément (1) étant réalisé sous forme de douille de réception (5) dans laquelle est prévu, à une extrémité, au moins un alésage de verrouillage radial (4), l'élément d'entraînement (7) étant placé sous forme de moteur électrique (8) dans la douille de réception (5), lequel entraîne, éventuellement par l'intermédiaire d'un arbre d'entraînement (10) ou directement, un système de rouleaux planétaires (11) ou un système de filets ou de tiges,
**caractérisé par le fait que**
une section intérieure (17) du premier élément (1) est réalisée de manière polygonale ou rectangulaire comme zone de guidage (15) pour le deuxième élément (2) des deux éléments (1, 2); et
une section extérieure (18) du deuxième élément (2) correspond au moins partiellement à la section intérieure (17) du premier élément (1) dans la zone de guidage (15) et est réalisée de manière polygonale ou rectangulaire.

2. Dispositif selon la revendication 1, **caractérisé par le fait qu'**à l'élément d'entraînement (7), au moteur électrique (8), à l'arbre d'entraînement (10) et/ou au système de rouleaux planétaires (11) ou au système de tiges est associé au moins un capteur de force (13) et/ou un capteur de trajet (13) qui communique par exemple par des systèmes de tiges avec l'élément d'entraînement (7), en particulier le moteur électrique (8), avec une unité électronique (6).

3. Dispositif selon l'une des revendications 1 ou 2, **caractérisé par le fait qu'**une section intérieure du deuxième élément (2) est réalisée sous forme d'alésage cylindrique qui est pourvu, dans sa surface d'enveloppe intérieure, d'un filet (21) qui vient en prise avec le système de rouleaux planétaires (11) ou le système de filets ou tiges se raccordant à l'arbre d'entraînement (10).

4. Dispositif selon l'une des revendications précédentes, **caractérisé par le fait que** du côté de l'extrémité le deuxième élément (2) présente au moins un alésage de verrouillage radial (4).

5. Dispositif selon l'une des revendications précédentes, **caractérisé par le fait qu'**il est placé au moins un joint ou au moins un élément d'étanchéité (22) entre les éléments (1,2).

6. Dispositif selon l'une des revendications précédentes, **caractérisé par le fait que** du côté opposé à la zone du côté d'extrémité (14) du premier élément (1) est placé de manière bidirectionnelle et sana contact un élément de transmission d'énergie et/ou de données (3), en particulier au moins une bobine pour la transmission inductive d'énergie et/ou de données qui communique avec l'élément d'entraînement (7) et/ou l'unité électronique (6).
